# EUROPEAN PATENT SPECIFICATION

(11) **EP 3 603 605 B1**
(45) Date of publication and mention of the grant of the patent: **28.09.2022**
(21) Application number: 18798674.0
(22) Date of filing: 10.05.2018
(51) Int. Cl.: A61J 3/00, G16H 10/00, A61J 7/00

(54) **DRUG DISPENSING DEVICE, CONTROL PROGRAM FOR DRUG DISPENSING DEVICE, AND RECORDING MEDIUM WITH RECORDED CONTROL PROGRAM**
ARZNEIMITTELABGABEVORRICHTUNG, STEUERPROGRAMM FÜR ARZNEIMITTELABGABEVORRICHTUNG UND AUFZEICHNUNGSMEDIUM MIT AUFGEZEICHNETEM STEUERPROGRAMM
DISPOSITIF DE DISTRIBUTION DE MÉDICAMENT, PROGRAMME DE COMMANDE POUR DISPOSITIF DE DISTRIBUTION DE MÉDICAMENT, ET SUPPORT D'ENREGISTREMENT AVEC PROGRAMME DE COMMANDE ENREGISTRÉ

(30) Priority: 12.05.2017 JP 2017095576
(43) Date of publication of application: 05.02.2020
(73) Proprietor: YUYAMA MFG. CO., LTD., Toyonaka-shi, Osaka 561-0841 (JP)
(72) Inventor: SHIBATA, Tomoyuki, Toyonaka-shi, Osaka 561-0841 (JP); MATSUURA, Takuya, Toyonaka-shi, Osaka 561-0841 (JP)
(74) Representative: Winter, Brandl - Partnerschaft mbB
(86) International application number: PCT/JP2018/018110
(87) International publication number: WO 2018/207868

(56) References cited:
- WO-A1-2011/052766
- WO-A1-2012/070643
- WO-A1-2014/112221
- JP-A- 2006 051 178
- JP-A- 2010 525 900
- JP-A- 2010 533 927
- US-A1- 2003 216 831
- US-A1- 2008 059 228
- US-A1- 2013 212 987
- US-A1- 2014 021 253
- US-B1- 8 271 128

## Description

### Technical Field

The present invention relates to a drug dispensing device configured to dispense a drug separately at different times of administration.

### Background Art

Hitherto, there has been known a drug dispensing device configured to separately dispense one dose of a prescribed drug (e.g., refer to JP 2010-5378 A). With the drug dispensing device, each dose of the prescribed drug is dispensed separately at an administration timing of "right after waking up", "after breakfast", "after lunch", "after supper", or "before sleeping", for example. The dispensed drug is provided to a patient under a state in which each dose of the dispensed drug is wrapped in a packaging paper sheet or one week's doses of the dispensed drug is packaged in a blister pack. There is an advantage in that it is possible to prevent a patient from missing a dose or prevent administration of a wrong drug, by packaging the drug for each dose of administration in this manner.

US 2008/0059228 A discloses a delivery module provided at a Patient's residence. The delivery module accommodates medication carriers, which includes a receptacle for holding individual, unit dose packages in a non-sequential fashion. The delivery module delivers unit dose package of medication that has been ejected from the medication carrier to a patient.

### Summary of Invention

### Technical Problem

It is desired that there be a system in which, after the drug dispensing device has dispensed a drug, when some problem is detected to have occurred in a specific lot at the time of manufacture of the drug, for example, it is possible to easily track patients to which the drug of that lot has been prescribed.

In view of the above-mentioned demand, the present invention has an object to provide a drug dispensing device having a function of managing manufacturing information on a drug dispensed to each patient.

### Solution to Problem

In order to achieve the above-mentioned object, a drug dispensing device according to claim 1 is provided. Advantageous embodiments are subject-matter of the dependent claims.

### Advantageous Effects of Invention

According to the above-mentioned configuration, it is possible to provide the drug dispensing device having the function of managing the manufacturing information on the dispensed doses of the drug. As a result, it is possible to easily track the dispensed drug when some inconvenience is detected to have occurred in the dispensed drug at a later date.

### Brief Description of Drawings

FIG. 1 is an external view of a drug dispensing device according to one embodiment of the present invention.
FIG. 2 is a perspective view of the drug dispensing device in a state in which an upper cover of a dispensing unit is opened and a drawer is drawn out toward a front side.
FIG. 3 is a top view of an appearance of a blister pack.
FIG. 4 is an external view of the drug dispensing device mounted with a computer and a barcode reader.
FIG. 5 is a block diagram for illustrating a schematic configuration of control of the drug dispensing device.
FIG. 6 is an explanatory diagram for illustrating an example of a cassette monitor screen.
FIG. 7 is a flow chart for illustrating a procedure of processing to be executed when a tablet cassette is refilled with a drug.
FIG. 8 is an explanatory diagram for illustrating an example of a tablet refilling details screen.
FIG. 9 is a flow chart for illustrating a procedure of drug dispensing processing.
FIG. 10 is an explanatory diagram for illustrating an example of a tablet manual-dispensing support screen.
FIG. 11 is an explanatory diagram for illustrating an example of a search screen for searching a history database.

### Description of Embodiments

In the present invention, the term "manufacturing information" means information enabling identification of a manufacturing time and/or a manufacturing location (e.g., manufacturing region, manufacturing factory, or manufacturing line) of the drug. For example, a lot number assigned to a container of a medication by a drug manufacturer or the like can be used as the manufacturing information. With this, for example, when, at a later date, some problem is detected to have occurred in a product manufactured at a specific time for some medication dispensed as a medication to be manually dispensed, or when, at a later date, some problem is detected to have occurred in a product manufactured at a specific factory, for example, it is possible to track patients to which the problematic medication has been dispensed, based on information stored in the storage.

According to the present invention, the worker inputs manufacturing information on a drug to be manually dispensed every time the worker dispenses the drug. With this, it is possible to completely store the manufacturing information also on a drug to be dispensed through manual-dispensing work.

According to the present invention, in the device configured to automatically dispense a tablet by using the tablet cassette, it is possible to completely store the manufacturing information on a medication to be dispensed from the tablet cassette .

In one embodiment, it is preferred that the tray be configured to receive a tray of a blister pack.

Further, in another embodiment, it is preferred that the drug dispensing device further include a dispensing unit configured to dispense, to an individual drug package, a drug input to each of the plurality of cells of the tray.

Now, referring to the drawings, a detailed description is given of an embodiment of the present invention. The same or corresponding parts are denoted by the same reference symbols in the drawings, and a description thereof is not repeated. In order to facilitate understanding of illustration, in the drawings referred to below, the configuration is simplified or schematic for illustration, or some of components are omitted. Further, a dimension ratio between components illustrated in each figure does not always reflect an actual dimension ratio.

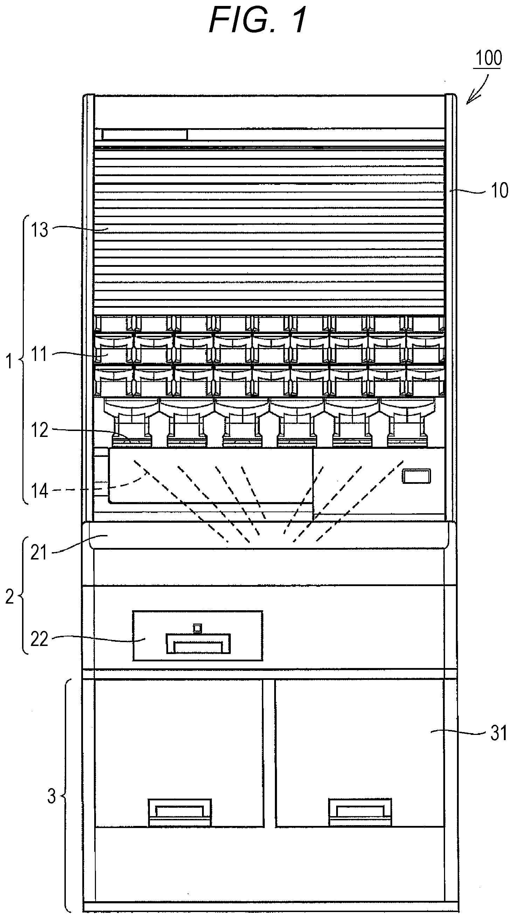

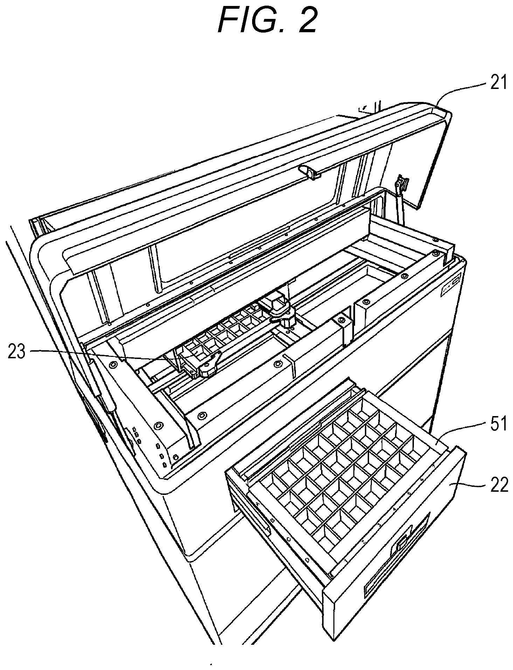

In the following embodiment, a description is given of a drug dispensing device configured to dispense a drug to a blister pack as an example. FIG. 1 is an external view of a drug dispensing device 100 according to an embodiment of the present invention. As illustrated in FIG. 1, the drug dispensing device 100 includes a tablet supply unit 1 provided in an upper half part of a frame 10. A dispensing unit 2 (refer to FIG. 2), which protrudes forward from the upper half part of the frame 10, is provided in a lower half part of the frame 10. A storage 3 for storing an unused blister pack and the like is provided in a further lower part of the frame 10. The tablet supply unit 1 can store tablet cassettes 11 and 12 as multiple stages of cassettes . The tablet supply unit 1 is opened or closed in a vertical direction by a shutter 13 provided on a front side thereof. FIG. 1 is an illustration of a state in which about half the shutter 13 is opened.

The tablet cassette 11 can dispense a required quantity of a predetermined specific type of tablet one by one (unit quantity) . The tablet cassette 12 is different from the tablet cassette 11 dedicated for a specific type of tablet in that the tablet cassette 12 can be refilled with any type of tablet as required to be used. However, similarly to the tablet cassette 11, the tablet cassette 12 can dispense only a required quantity of tablets one by one (unit quantity). In order to dispense a specific type of tablet, each tablet cassette 11 is designed to have a discharge port of tablets that has a shape and size adjusted to the specific type of tablet. The tablet cassette 12 is designed to have a discharge port having a size or the like that can be adjusted depending on a drug to be dispensed, and the tablet cassette 12 is refilled with a required type of drug for use every time the drug is required. The tablets dispensed from the tablet cassettes 11 and 12 pass through a hopper 14 (indicated by the broken lines in FIG. 1) provided inside the tablet supply unit 1, and are sent to the dispensing unit 2. Tablets of a drug for which the tablet cassettes 11 and 12 cannot be used or a drug for which use of the tablet cassettes 11 and 12 is inefficient or inappropriate, for example, are dispensed by manual-dispensing work described later.

In other words, a medication other than a specific type of medication to be dispensed from the tablet cassette 11 is dispensed by any one of methods of (1) dispensing the medication through manual-dispensing work by a pharmacist checking a prescription, and (2) refilling the tablet cassette 12 with the medication and dispensing the medication from the tablet supply unit 1. The pharmacist can determine which one of the methods (1) and (2) is to be used in consideration of a characteristic and use condition of the medication, and work efficiency. Thus, herein, a medication other than the specific type of medication to be dispensed from the tablet cassette 11 is referred to as "medication to be manually dispensed". Further, for example, when the tablet cassette 11 becomes empty near the end of dispensing work, remaining several tablets may be dispensed by manual-dispensing work at higher work efficiency. In this manner, when a medication that is normally dispensed from the tablet cassette 11 is dispensed by manual-dispensing work, this medication is also referred to as "medication to be manually dispensed".

A drug packaging device that uses a tablet cassette to be refilled with any type of tablet to be used, for example, the tablet cassette 12, is described in detail in a prior application (PCT/JP 2016/054349 and WO 2016/136523) filed by the applicant of the present invention, and the details of the prior application are incorporated herein by reference.

The dispensing unit 2 (manually-dispensed medication processor) includes an upper cover 21 and a drawer 22. FIG. 2 is a perspective view of the dispensing unit 2 in a state in which the upper cover 21 is opened and the drawer 22 is drawn out toward a front side. As illustrated in FIG. 2, the dispensing unit 2 includes a divided tray 23 (details thereof are described later). Further, the drawer 22 receives a tray 51 of the blister pack 5 under a state in which the drawer 22 is drawn out toward the front side. That is, the drawer 22 includes a placement part adjusted to the cell shape of the tray 51.

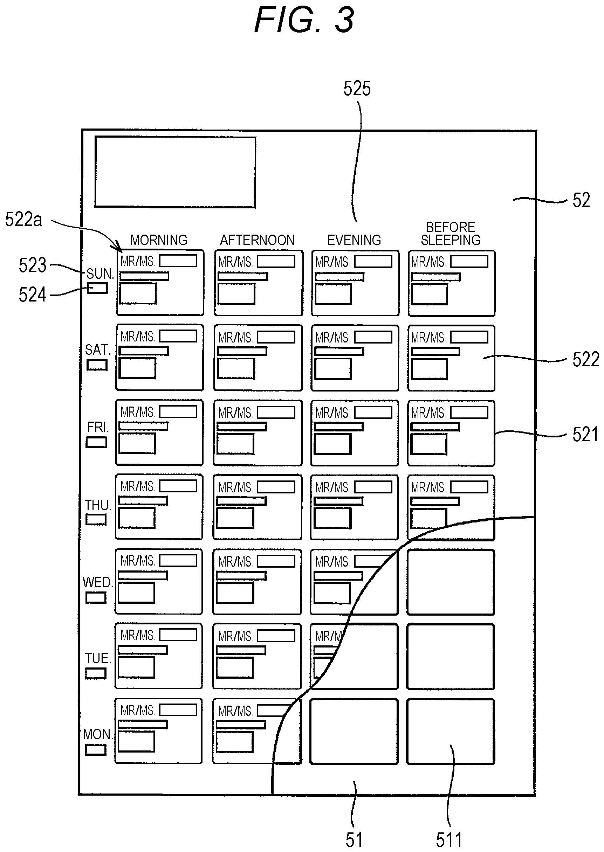

Now, referring to FIG. 3, a description is given of the blister pack 5. FIG. 3 is a top view of an appearance of the blister pack 5. As illustrated in FIG. 3, the blister pack 5 has a configuration in which a cover sheet 52 is attached to an opening surface of the tray 51 under a state in which tablets (not shown) are input to a plurality of cells 511 of the tray 51. The tray 51 is made of transparent resin so that whether the dispensed drug follows the prescription can be checked from the back surface of the blister pack 5. In FIG. 3, for the sake of description, a state in which a part of the cover sheet 52 is cut out is illustrated. However, in the actual blister pack 5, the entire opening surface of the tray 51 is completely sealed by the cover sheet 52.

The blister pack 5 illustrated in FIG. 3 includes 28 cells 511 corresponding to a total of 28 administration times, which are four times of "morning", "afternoon", "evening", and "before sleeping" per day multiplied by one week of from Sunday to Monday.

In the cover sheet 52, a removal portion 522 enclosed by a break line 521 is provided so as to fit an opening portion of each of the 28 cells 511. An adhesive is applied to the surrounding of the removal portion 522 on the back surface of the cover sheet 52, and the cover sheet 52 can be attached to the tray 51 by this adhesive. A release paper (not shown) is provided on the back surface of the cover sheet 52 before being attached to the tray 51 so as to protect the adhesive. A patient name 522a or the like is printed on each removal portions 522 of the cover sheet 52. Any other pieces of information such as an administration time and a dose may be printed in addition to the patient name. Further, a seven-days display 523, a date display 524 corresponding thereto, and an administration display 525 are printed on the margin of the cover sheet 52.

At the time of administration, for each of four administration times of the printed day, a patient (or nurse, for example) pushes the corresponding removal portion 522 by his or her finger, for example, to break the break line 521, and takes a tablet out of the corresponding cell 511. In this manner, tablets are stored separately for each administration time, and the day and date are printed. Thus, it is possible to prevent wrong drug administration. Further, the removal portion 522 corresponding to completed administration is broken, and thus it is possible to easily check missing of a dose.

Now, referring back to FIG. 2, a description is further given of the dispensing unit 2. As described above, the dispensing unit 2 includes the divided tray 23 inside the upper cover 21. The divided tray 23 includes the same number of cells as the number corresponding to the number of rows × the number of columns of the cells 511 of the tray 51 of the blister pack 5. Further, the divided tray 23 is configured to move in three-axis directions, namely, XYZ directions inside the dispensing unit 2 by being driven by a motor, for example. The divided tray 23 moves in the XY directions with respect to a tablet discharge port of the hopper 14 of the tablet supply unit 1 so that a drug delivered from the hopper 14 is stored in a cell corresponding to the cell 511 to which the drug is to be input.

The bottom surface of the cell of the divided tray 23 is configured to be opened and closed. When all the drugs to be dispensed from the tablet supply unit 1 are dispensed to the divided tray 23 based on prescription data, the divided tray 23 moves toward the upper side of the tray 51 inside the dispensing unit 2, and opens the shutter of the bottom surface of the cell. With this, the drug of each cell of the divided tray 23 is delivered to the corresponding cell 511 of the tray 51.

The configuration and operation of the divided tray 23 are described in detail in a prior application (PCT/JP 2011/077179 and WO 2012/070643) filed by the applicant, and the details of the prior application are incorporated herein by reference.

In this embodiment, a description has been given of the exemplary configuration including the divided tray 23 inside the dispensing unit 2 separately from the tray 51 of the blister pack 5. However, the divided tray 23 is not indispensable, and can be omitted. That is, there may be provided a configuration of directly dispensing a drug to the tray 51 from the tablet supply unit 1 by omitting the divided tray 23.

Further, when the prescription data includes a tablet that cannot be dispensed from the tablet cassettes 11 and 12 of the tablet supply unit 1, the drug is dispensed to the tray 51 by manual-dispensing work under a state in which the drawer 22 in which the tray 51 is set is drawn out. When the prescription data includes both a drug to be dispensed from the tablet supply unit 1 and a drug to be dispensed by manual-dispensing work, a worker (pharmacist) can freely select which drug is to be dispensed first.

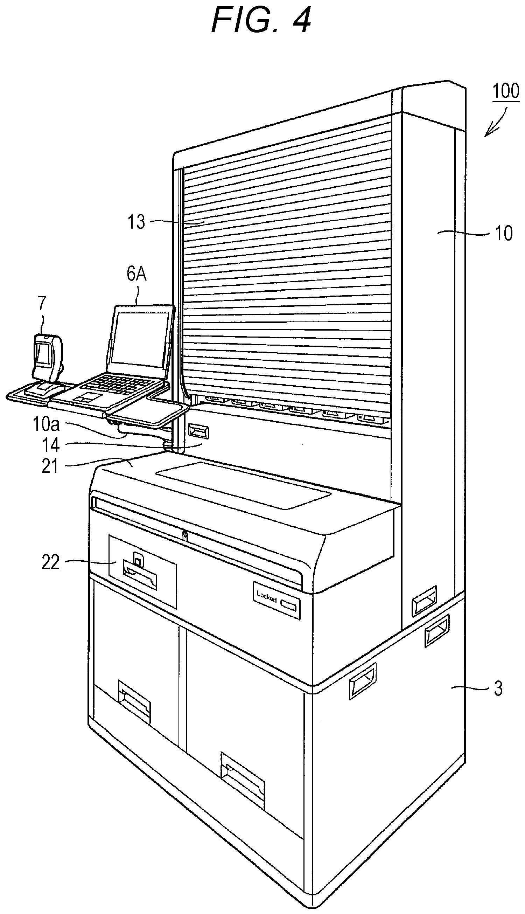

As illustrated in FIG. 4, the drug dispensing device 100 includes a support stand 10a mounted to the frame 10, and a computer 6A (corresponding to control unit 6 described later) and a barcode reader 7 can be mounted on the support stand 10a. The worker can perform input by operating, for example, a keyboard or a touch panel of the computer 6A while looking at a work support screen displayed on the monitor of the computer 6A. In FIG. 4, a laptop computer is illustrated as an example of the computer 6A, but a tablet computer or a smartphone may be used, for example. The computer 6A and the drug dispensing device 100 may communicate to/from each other in a wired manner or a wireless manner . The barcode reader 7 is used for reading a barcode from the container of the drug, for example. Any other reading means such as a QR code (trademark) reader or an IC tag reader may be included instead of the barcode reader 7 or in addition to the barcode reader 7 depending on the characteristic of a code to be read.

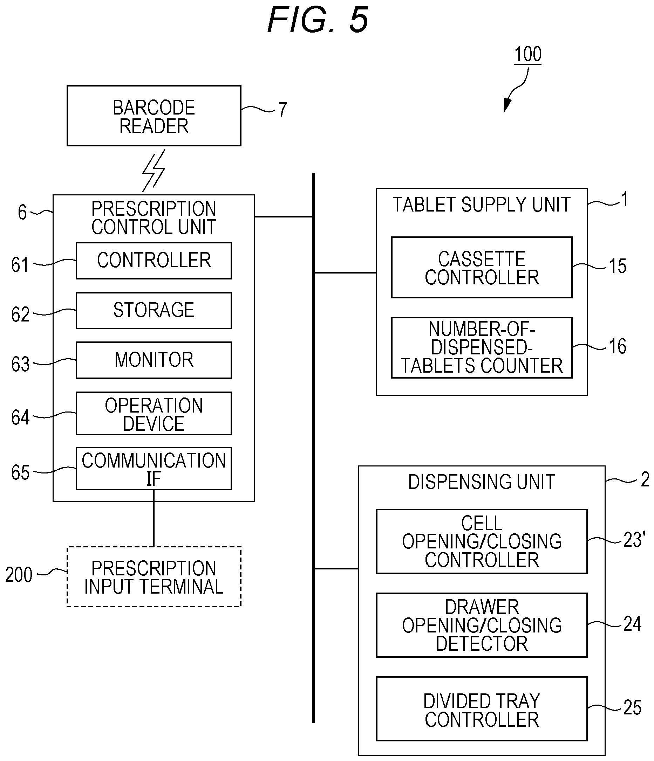

FIG. 5 is a block diagram for illustrating a schematic configuration of control of the drug dispensing device 100. The drug dispensing device 100 includes the control unit 6 in addition to the tablet supply unit 1 and the dispensing unit 2 described above. In this embodiment, the computer 6A implements the function of the control unit 6. However, the configuration is not limited thereto, and a processor, a memory, or other components inside the drug dispensing device 100 may function as all or a part of the control unit 6. Now, a description is given of a functional configuration of the control unit 6, the tablet supply unit 1, and the dispensing unit 2.

### [Control Unit 6]

The control unit 6 is configured to read prescription data, display an operation screen, and receive an instruction given by the worker. Thus, the control unit 6 includes, for example, a controller 61, a storage 62, a monitor 63, an input device 64 (operation device 64), and a communication IF 65. In this embodiment, those components are implemented by the computer 6A.

The monitor 63 can be implemented by any display. The input device 64 can be implemented by, for example, a keyboard and a touch panel. The input device 64 may be configured to receive input of a voice. Further, the control unit 6 may include a speaker, and may be capable of outputting a voice.

The controller 61 includes, for example, a CPU, a RAM, a ROM, and an EEPROM (which are not shown) . The controller 61 uses the CPU to execute various kinds of programs stored in advance in the ROM, EEPROM, or the storage 62, for example. The RAM and the EEPROM are used as a temporary memory (work area) at the time of execution of various kinds of processing by the CPU. The controller 61 may be an integrated circuit such as an ASIC or a DSP.

The storage 62 is a hard disk device or a solid state drive (SSD) . The storage 62 stores in advance a program for causing the CPU of the controller 61 to execute predetermined processing.

The program is stored in a computer-readable recording medium such as a CD, a DVD, or a semiconductor memory, and is read from the recording medium by a reading device, for example, a disk drive (not shown) to be installed into the storage 62. The present invention can also be regarded as an invention of the computer-readable recording medium having recorded thereon the program.

Further, the storage 62 stores various kinds of databases such as a medication master, a patient master, an accommodated medication master, a worker master, and a pharmacy master. The controller 61 can update various kinds of databases stored in the storage 62 based on data read by the reading device, for example, a disk drive (not shown), from the recording medium such as a CD, a DVD, or a semiconductor memory. Further, the controller 61 can also change the details of the various kinds of databases in accordance with an operation of the operation device 64 performed by the user.

The medication master stores information on each medication such as a medicine ID, a medication code, a medication name, a JAN code (or RSS code), a medicine bottle code, a category (dosage form: a powdered medicine, a tablet, a liquid medicine, and a medicine for external use), a size (height and width) of a tablet, a specific gravity, a medication type (e.g., an ordinary medicine, a poison, a narcotic, a powerful drug, an antipsychotic drug, or a curative medicine), an incompatibility, an excipient, and a precaution. Further, the medication master may store an image of a tablet. The patient master stores information on a patient such as a patient ID, a name, a sex, an age, a medical history, a prescribed medicine history, family information, a clinical department, a ward, and a hospital room. The pharmacy master stores information on a pharmacy such as a pharmacy name, the name of a pharmacist, and the ID of the pharmacist.

The accommodated medication master stores cassette identification information (cassette number) assigned to each of the tablet cassettes 11 and 12 of the tablet supply unit 1, and information (e.g., a medication code, a medication name, a maximum accommodated quantity, a reference refill quantity, a used quantity, a stock quantity, a serial number (manufacturing information), and an expiry date) on a medication stored in each cassette. The maximum accommodated quantity is the maximum number of tablets that can be stored in a cassette. The reference refill quantity is a reference number of medications for determining refill of a medication, and when the stock quantity falls below the reference refill quantity, medications are refilled to a cassette. The used quantity is a quantity of dispensed medications. The stock quantity is the remaining number of medications inside a cassette. The serial number is information assigned to a medication by a medication manufacturer to identify, for example, a manufacturing location, a manufacturing time, and a manufacturing line of a medication, and is a lot number, for example. The expiry date is a date set by the medication manufacturer as the expiry date of a medication.

Information of the accommodated medication master is registered by the controller 61 in accordance with an operation of the operation device 64 performed by the user at the time of initial setting of the drug dispensing device 100. Further, every time the tablet cassettes 11 and 12 are refilled with tablets during usage of the drug dispensing device 100, information of the accommodated medication master is updated for the cassette supplied with tablets. The operation to be performed at the time of refilling tablets is described in detail later.

Further, the storage 62 also stores a history database that stores a history of dispensing drugs in the drug dispensing device 100 at least temporarily. The history database records which drug is prescribed to which patient at what time. That is, the history database records information (e.g., medication ID and medication name) for identifying a drug dispensed to each patient in association with information (e.g., patient ID and name) for identifying a patient. Further, the history database also records a serial number (e.g., lot number) and expiry date of each dispensed drug. In this embodiment, the history database is provided in the storage 62 of the control unit 6 (computer 6A). However, the history database may be provided in the storage device other than the control unit 6. Further, the details of the history database provided in the storage 62 may periodically be saved to a storage device outside the drug dispensing device 100.

The work support screen is displayed on the monitor 63 by the controller 61 executing control based on prescription data and input performed by the worker.

The operation device 64 is operation means for receiving an operation performed by the user, such as a keyboard, a mouse, and a touch panel, and inputs to the controller 61 an operation signal corresponding to the operation performed by the user. The communication IF 65 is a communication interface for connecting the drug dispensing device 100 to an external prescription input terminal 200 via a communication network. The communication IF 65 is configured to execute data communication to/from a host system, for example, the prescription input terminal 200. The prescription input terminal 200 is, for example, an electronic health record provided in a hospital or a healthcare institution for the elderly, or a dispensing management system provided in a pharmacy inside or outside the hospital. The communication IF 65 also includes a wireless communication interface, for example, a wireless communication card configured to execute wireless data communication to/from various kinds of wireless communication devices, for example, the barcode reader 7.

The communication IF 65 is configured to acquire prescription data from the prescription input terminal 200, and input the acquired prescription data to the controller 61. For example, the communication IF 65 monitors whether prescription data is stored in a predetermined storage area of storage means provided in the prescription input terminal 200, and when the prescription data is stored in the predetermined storage area, the communication IF 65 reads the prescription data from the predetermined storage area. The communication IF 65 may receive the prescription data transmitted from the prescription input terminal 200.

### [Tablet Supply Unit 1]

As illustrated in FIG. 5, the tablet supply unit 1 includes a cassette controller 15 and a number-of-dispensed-tablets counter 16. The cassette controller 15 controls the operation of dispensing tablets from the tablet cassettes 11 and 12 in accordance with an instruction from the control unit 6 that is based on the prescription data. An RFID tag (not shown), for example, are assigned to each of the tablet cassettes 11 and 12 so that the tablet cassettes 11 and 12 store, for example, cassette identification information for identifying each cassette and drug information for identifying the accommodated drug. A tag reader for reading RFID tags of the tablet cassettes 11 and 12 is provided in the tablet supply unit 1, and selects a cassette accommodating a target tablet from the tablet cassettes 11 and 12 in accordance with the prescription data. The selected cassette is controlled by the cassette controller 15 to be moved to a reception port of the hopper 14 by, for example, a motor mechanism inside the tablet supply unit 1, and a required quantity of tablets are dispensed in accordance with the prescription data. The number-of-dispensed-tablets counter 16 counts the number of dispensed tablets, and transmits the number of dispensed tablets to the control unit 6 together with cassette identification information on the tablet cassette that has dispensed a drug. The control unit 6 updates the stock quantity in the accommodated medication master of the storage 62 based on the number of dispensed tablets counted by the number-of-dispensed-tablets counter 16. With this, the stock quantity of tablets inside each cassette in the accommodated medication master is updated to the latest state.

### [Dispensing Unit 2]

The dispensing unit 2 includes a cell opening/closing controller 23' , a drawer opening/closing detector 24, and a divided tray controller 25. The cell opening/closing controller 23' is configured to control opening/closing of the shutter of the bottom surface of a cell of the divided tray 23. When the drawer 22 is drawn out, the drawer opening/closing detector 24 detects this fact, and outputs a detection signal to the control unit 6. The divided tray controller 25 drives the motor or other components inside the dispensing unit 2 in accordance with an instruction from the control unit 6 that is based on the prescription data, to thereby control the motion of the divided tray 23.

Next, a description is given of an operation of the drug dispensing device 100 having the above-mentioned configuration. First, a description is given of an operation to be executed when the tablet cassettes 11 and 12 are refilled with tablets before the tablets are started to be dispensed.

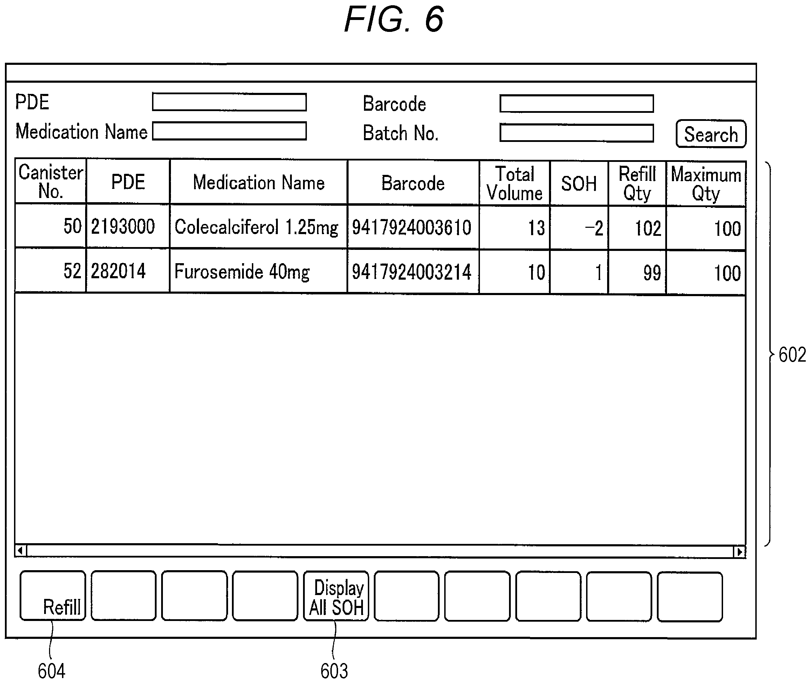

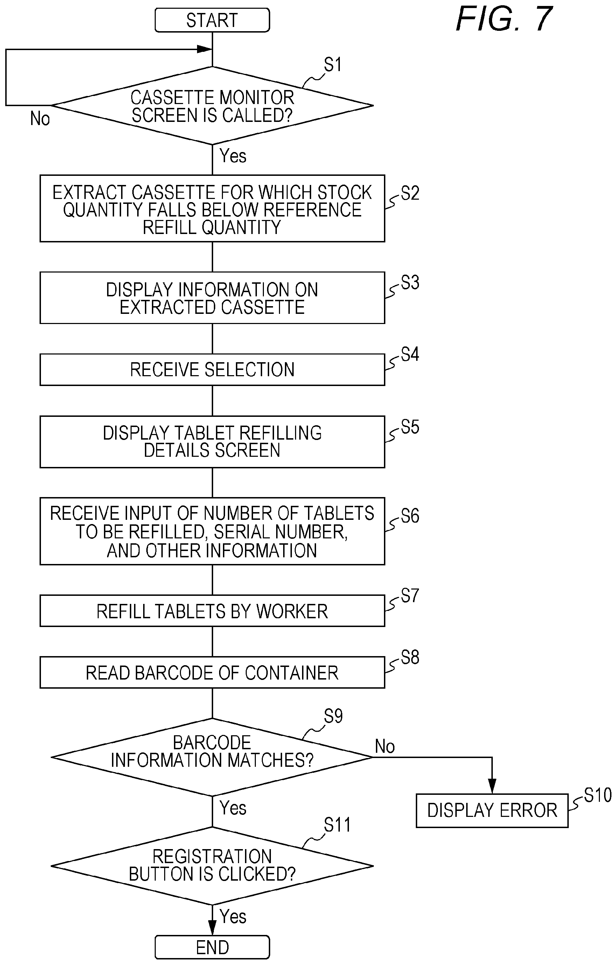

FIG. 6 is an explanatory diagram for illustrating an example of the cassette monitor screen to be displayed on the monitor 63 of the computer 6A. The cassette monitor screen is a screen for displaying information on drugs accommodated in the drug cassettes 11 and 12. When the worker performs an operation of moving from the main menu screen to the cassette monitor screen ("Yes" in Step S1 of FIG. 7), the controller 61 refers to the accommodated medication master of the storage 62 to extract a tablet cassette for which the stock quantity falls below the reference refill quantity (Step S2), and displays, on a cassette medication display area 602, a list of pieces of information on the extracted cassette (Step S3) .

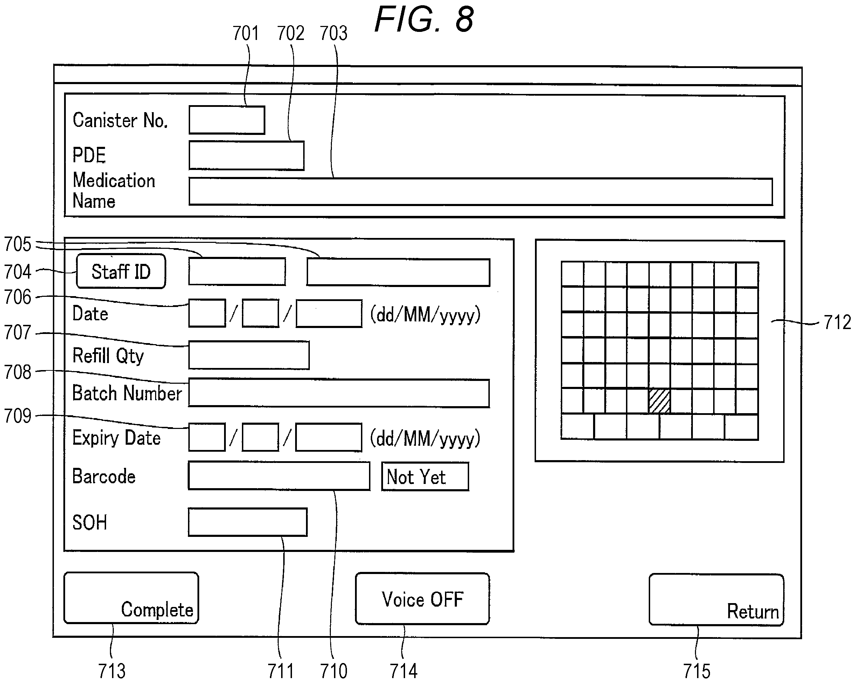

Then, when the worker has selected a cassette to be refilled from among cassettes displayed on the cassette medication display area 602 and clicked a refilling start button 604, the controller 61 receives this operation (Step S4), and switches display on the monitor 63 to such a tablet refilling details screen as illustrated in FIG. 8 (Step S5).

The tablet refilling details screen illustrated in FIG. 8 includes a cassette number display field 701, a medication code display field 702, a medication name display field 703, a refilling person selection screen display button 704, a refilling person name display button 705, a work date input field 706, a refill quantity input field 707, a serial number input field 708, an expiry date input field 709, a barcode input field 710, a stock quantity display field 711, a cassette position display field 712, a registration button 713, a voice off button 714, and a return key 715.

The number of a cassette to be refilled is displayed in the cassette number display field 701. A medication code of the drug to be refilled is displayed in the medication code display field 702. A medication name of the drug to be refilled is displayed in the medication name display field 703. The medication code and the medication name are acquired from the accommodated medication master. When the refilling person selection screen display button 704 is clicked, another screen for selecting a worker in charge of refilling work is displayed. The worker is registered in advance in the storage 62, and a worker who is not registered yet can be added. The refilling person name display button 705 displays an ID or name of the selected worker. The work date input field 706 is a field for inputting a date on which refilling work is performed.

The refill quantity input field 707 is a field for the worker to input the quantity of drugs to be refilled. The serial number input field 708 is a field for inputting the serial number of the drug to be refilled. When the tablet refilling details screen is opened, the serial number stored in the accommodated medication master is acquired and displayed on the tablet refilling details screen. The expiry date input field 709 is a field for inputting an expiry date of the drug to be refilled. When the tablet refilling details screen is opened, the expiry date stored in the accommodated medication master is acquired and displayed on the expiry date input field 709. The barcode input field 710 is a field for inputting the barcode of the drug to be refilled. When the tablet refilling details screen is opened, the barcode stored in the accommodated medication master is acquired and displayed on the tablet refilling details screen.

The stock quantity stored in the accommodated medication master for the drug is displayed in the stock quantity display field 711. The position of the tablet cassette is displayed in the cassette position display field 712 by flashing of a red color. In FIG. 8, display of flashing of a red color is represented by hatching. The registration button 713 is a button to be clicked when input is complete. The voice off button 714 is a button for turning off voice output. The return key 715 is a button for returning to the cassette monitor screen illustrated in FIG. 6.

The worker inputs the number of tablets to be refilled in the refill quantity input field 707 of the tablet refilling details screen by using a keyboard, for example (Step S6). Further, when the serial number and expiry date of a tablet to be refilled are changed based on information displayed in the serial number input field 708 and the expiry date input field 709, new information is input on those fields.

When input is complete, the worker draws out the tablet cassette to be refilled with drugs, from the tablet supply unit 1. At this time, the worker can easily check the position of the cassette in the tablet supply unit 1 by looking at the display of the cassette position display field 712. Then, after refilling the drawn tablet cassette with tablets, the worker sets the tablet cassette at the original position (Step S7).

Next, the worker causes the barcode reader 7 to read a barcode assigned to a container storing the refilled drug (Step S8). The barcode assigned to the container contains information for identifying the medication, and thus the controller 61 extracts the medication ID and medication name from the medication master of the storage 62 based on the read information on the barcode, and compares those pieces of information with information of the accommodated medication master, to thereby check whether a correct drug is refilled (Step S9). When the barcode information matches the information of the accommodated medication master ("Yes" in Step S9) and the worker has clicked the registration button 713, the refilling processing is complete (Step S11). In Step S9, when the barcode information does not match the information of the accommodated medication master, the controller 61 outputs an error message to the monitor 63 (Step S10).

The above-mentioned refilling processing is usually executed before starting the work of dispensing a drug. When the stock quantity of the drug in any one of the tablet cassettes 11 and 12 falls below the reference refill quantity during the work of dispensing a drug, the controller 61 detects this fact, and displays the tablet refilling details screen for that drug on the monitor 63. Further, the controller 61 outputs, from a speaker, a voice or warning sound, for example, "Please refill tablets". Then, when the worker has clicked the voice off button 714, the controller 61 stops output of a voice, for example. Then, after the processing of refilling the tablet cassette, which is short of tablets, with tablets in accordance with the same procedure as that of Step S6 to Step S11 of FIG. 7, the dispensing processing is resumed.

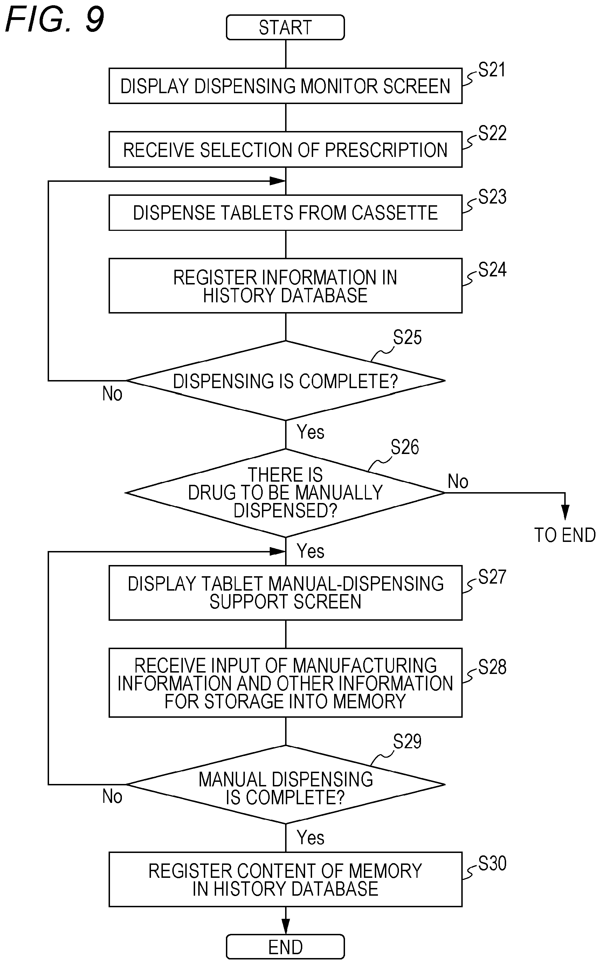

Now, referring to the flow chart of FIG. 9, a description is given of the procedure of the drug dispensing processing to be executed by the drug dispensing device 100.

When the worker has selected "prescription" for executing the drug dispensing processing on the screen displayed on the monitor 63, the controller 61 displays a list (not shown) of prescriptions to be processed on the monitor 63 (Step S21). The prescription data is data on a prescription for one patient, and is input from the prescription input terminal 200 to be read into the drug dispensing device 100. Then, when the worker has selected any one of the prescriptions, the controller 61 receives the selection (Step S22), and controls the dispensing unit 2 based on prescription data on the selected prescription, to thereby dispense the drug included in the prescription data from the tablet cassettes 11 and 12 (Step S23). Further, every time one type of drug included in the prescription data is dispensed, the controller 61 extracts, from the accommodated medication master, the serial number and expiry date of the drug together with information for identifying the dispensed drug (e.g., medication ID and medication name), and registers those pieces of information in the history database of the storage 62 in association with information (e.g., patient ID and patient name) for identifying the patient and prescription date, for example (Step S24). Through the registration of those pieces of information in the history database, for example, when some inconvenience is detected to have occurred at the time of manufacture of the drug, it is possible to use the history database to easily search for when the drug of a manufactured lot that has incurred the inconvenience is prescribed to which patient.

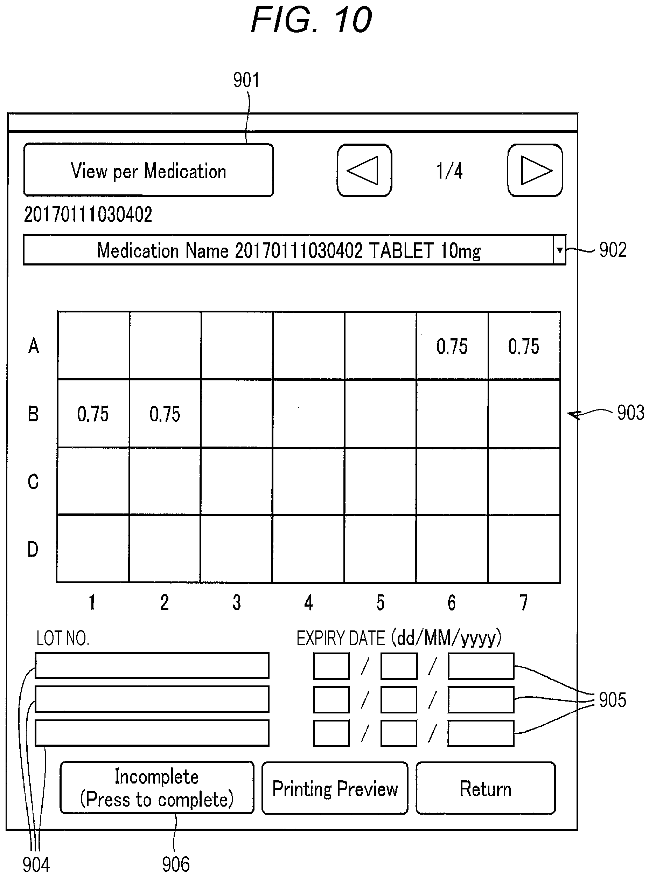

Among the drugs included in the prescription data, when all the drugs that can be dispensed from the tablet cassettes 11 and 12 have been dispensed and registered in the history database ("Yes" in Step S25), the controller 61 refers to the prescription data to determine whether there is a drug to be dispensed through manual dispensing (Step S26). When the prescription data includes a drug to be dispensed through manual dispensing ("Yes" in Step S26), the controller 61 displays on the monitor 63 such a tablet manual-dispensing support screen as illustrated in FIG. 10 (Step S27). When there is no drug to be dispensed through manual dispensing ("No" in Step S26), the controller 61 ends the processing.

The tablet manual-dispensing support screen illustrated in FIG. 10 includes, for example, a display switching button 901, a medication name display field 902, a manual-dispensing instruction area 903, a serial number input field 904, a validity date input field 905, and a manual-dispensing completion button 906. Every time the display switching button 901 is clicked, the display state of the tablet manual-dispensing support screen is switched to "per medication", "only manual dispensing", and "all medications" in order. Further, a specific display state of the screen is displayed on the display switching button 901. An exemplary screen of FIG. 10 has the display state of "per medication (view per medication)". Medications included in the prescription data can be displayed on the medication name display field 902 as a pull-down list, and a medication name selected by the worker from the pull-down list is displayed.

Squares representing the plurality of cells 511 of the tray 51 are schematically displayed on the manual-dispensing instruction area 903 in the same arrangement as the direction in which the tray 51 is set in the drawer 22. As illustrated in FIG. 10, the quantity (per 3/4 tablet in the example of FIG. 10) of drugs to be input is displayed at the position of a cell to which the drug is to be input on the manual-dispensing instruction area 903. In this manner, it is possible to prevent an error in the manual-dispensing work by displaying the quantity of drugs to be input on the square of the tray 51 to which the drug is to be input.

Light emitting elements, for example, LEDs, may be mounted to the respective plurality of cells 511 of the tray 51 in the drawer 22, and an LED corresponding to the cell 511 to which the drug is to be input may be flashed based on the prescription data. The light emitting element, for example, an LED, may be mounted to emit light at the periphery of the cell 511, or the light emitting element may be mounted to cause the entire cell 511 to emit light.

The serial number input field 904 is a field for the worker to input the serial number (e.g., lot number) of a drug to be manually dispensed. The validity date input field 905 is a field for the worker to input the validity date of the drug to be manually dispensed. The worker inputs the serial number and validity date described in the container in the serial number input field 904 and the validity date input field 905, respectively, before the drug to be manually dispensed is taken out from the container and dispensed to the tray 51 (the worker may input the serial number and validity date after the drug is dispensed).

The manual-dispensing completion button 906 is a button to be clicked by the worker every time manual-dispensing work of one type of drug is complete. During the manual-dispensing work, "incomplete" is displayed on the manual-dispensing completion button 906, and the display is switched to "complete" after the manual-dispensing work is complete and this button is clicked. The manual-dispensing completion button 906 is set to the "complete" state, to thereby enable a drug to be manually dispensed next to be selected in the pull-down list of the medication name display field 902. Further, there may preferably be provided a configuration in which, when the manual-dispensing work is complete and this button is clicked, an error message is output as long as pieces of data are not input in the serial number input field 904 and the expiry date input field 905.

In the example illustrated in FIG. 10, three sets (namely, three boxes) of the serial number and expiry date can be input for one drug in the serial number input field 904 and the expiry date input field 905. However, there may be any number of fields that can be input.

The serial number and expiry date input in the serial number input field 904 and the expiry date input field 905 for one drug on the tablet manual-dispensing support screen are temporarily stored in the memory inside the control unit 6 in association with information (e.g., medication ID and medication name) for identifying the drug (Step S28). Then, when it is determined that the manual-dispensing work is complete for all the drugs to be manually dispensed ("Yes" in Step S29), the controller 61 reads the serial number and expiry date stored in the memory to store the serial number and expiry date into the history database of the storage 62 in association with information (e.g., patient ID and patient name) for identifying the patient and the prescription date, for example (Step S30). Through the registration of those pieces of information in the history database, for example, when, at a later date, some inconvenience is detected to have occurred at the time of manufacture of the drug to be manually dispensed, it is possible to use the history database to easily search for when the drug of a manufactured lot that has incurred the inconvenience is prescribed to which patient.

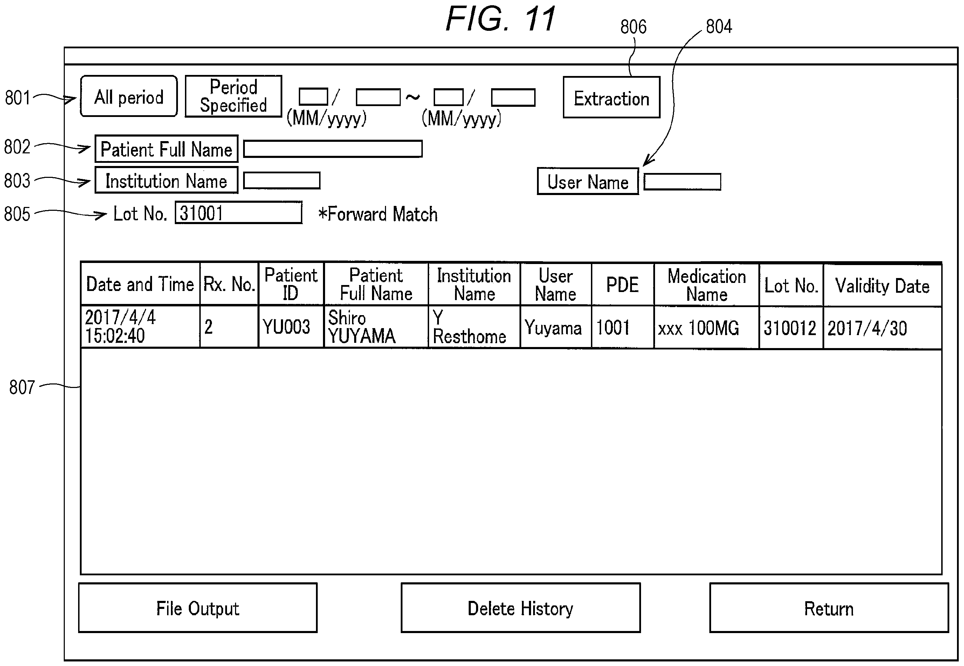

FIG. 11 is an illustration of an example of a search screen for searching the history database. As illustrated in FIG. 11, the search screen of the history database includes a period specification field 801 for specifying a range of a period to be searched, a patient specification field 802 for specifying a patient name, an institution specification field 803 for specifying an institution name, a worker specification field 804 for specifying a worker name, and a serial number specification field 805 for specifying a serial number. Further, when desired search conditions are specified on those fields and a search start button 806 is clicked, the controller 61 displays the search result in the search result display field 807 by searching the history database in accordance with specified search conditions.

In the example of FIG. 11, "31001" is input in the serial number specification field 805 as a search key. The controller 61 extracts a record including a serial number matching (forward match) the search key from the history database, and displays the record in the search result display field 807. In the example of FIG. 11, the record displayed in the search result display field 807 as the search result includes a prescription date ("Date and Time"), a prescription number ("Rx. No."), a patient ID ("Patient ID"), a patient name ("Patient Full Name"), an institution name ("Institution Name"), a worker name ("User Name"), a medication code ("PDE"), a medication name ("Medication Name"), a serial number ("Lot No."), and a validity date ("Validity Date").

As described above, according to the drug dispensing device 100 of the above-mentioned embodiment, every time a drug is dispensed based on the prescription for one patient, history information on which drug is prescribed to the patient at what time is registered in the history database. This history information includes information on the serial number and expiry date for each dispensed drug. With this, it is possible to check which drug is prescribed to which patient at a later date by referring to the history database. Further, it is also possible to search for the serial number of the dispensed drug, and thus it is possible to easily track to which patient the drug is dispensed when some inconvenience is detected in the dispensed drug at the time of manufacture of the drug.

### [Modification Example]

In the above, a specific description has been given of the embodiment of the present invention. However, the embodiment of the present invention is not limited to the mode described above, and various kinds of changes can be made thereto.

For example, in the above-mentioned embodiment, the description has been given of the drug dispensing device configured to dispense a tablet to the blister pack as an example. However, the mode of dispensing the drug is not limited to the blister pack. For example, the present invention can also be applied to the drug dispensing device including a dispensing unit configured to individually dispense one dose of tablets to a packaging paper sheet. Further, the present invention can also be applied to the drug dispensing device configured to dispense not only a tablet but also a powder medicine, for example.

Further, in the above-mentioned embodiment, the validity date is input together with the manufacture information (e.g., lot number) on the medication. However, input of the validity date is not indispensable.

Further, a description has been given of an exemplary configuration in which the storage 62 of the control unit 6 stores various kinds of masters and the history database. However, there may be provided a configuration in which the storage outside the control unit 6 stores various kinds of masters and the history database via a communication IF and an external network, for example.

A part or all of the processing described in the above-mentioned embodiment may be implemented by a program. In this case, a part or all of each processing procedure is executed by, for example, a central processing unit (CPU), a microprocessor, or a processor in the computer. A program for executing each processing procedure is stored in a storage device such as a hard disk drive or a ROM, and is read into the ROM or RAM for execution. The storage device (storage medium) is a non-transitory tangible device, and for example, a tape, a disk, a card, a semiconductor memory, and a programmable logical circuit can be used as the storage device.

Each processing procedure described in the above-mentioned embodiment may be implemented by hardware, or may be implemented by software (including a case of implementation through use of an operating system (OS), middleware, or a predetermined library). Further, each processing procedure described in the above-mentioned embodiment may be implemented by mixed processing of software and hardware. It is to be understood that, when display processing to be executed by the display apparatus in the above-mentioned embodiment is implemented by hardware, the timing for executing each processing procedure is required to be adjusted. In the above-mentioned embodiment, for the sake of convenience of description, details of adjustment of the timings of various kinds of signals accompanying an actual hardware design are omitted.

### Reference Signs List

- 1: tablet supply unit
- 15: cassette controller
- 16: number-of-dispensed-tablets counter
- 2: dispensing unit (manually-dispensed medication processor)
- 23': cell opening/closing controller
- 24: drawer opening/closing detector
- 25: divided tray controller
- 6: prescription control unit
- 61: controller
- 62: storage
- 63: monitor
- 64: input device
- 65: communication IF
- 6A: computer
- 7: barcode reader
- 100: drug dispensing device
- 200: prescription input terminal
Further, the present invention can also be applied to the drug dispensing device configured to dispense not only a tablet but also a powder medicine, for example.

Further, in the above-mentioned embodiment, the validity date is input together with the manufacture information (e.g., lot number) on the medication. However, input of the validity date is not indispensable.

Further, a description has been given of an exemplary configuration in which the storage 62 of the control unit 6 stores various kinds of masters and the history database. However, there may be provided a configuration in which the storage outside the control unit 6 stores various kinds of masters and the history database via a communication IF and an external network, for example.

A part or all of the processing described in the above-mentioned embodiment may be implemented by a program. In this case, a part or all of each processing procedure is executed by, for example, a central processing unit (CPU), a microprocessor, or a processor in the computer. A program for executing each processing procedure is stored in a storage device such as a hard disk drive or a ROM, and is read into the ROM or RAM for execution. The storage device (storage medium) is a non-transitory tangible device, and for example, a tape, a disk, a card, a semiconductor memory, and a programmable logical circuit can be used as the storage device.

Each processing procedure described in the above-mentioned embodiment may be implemented by hardware, or may be implemented by software (including a case of implementation through use of an operating system (OS), middleware, or a predetermined library). Further, each processing procedure described in the above-mentioned embodiment may be implemented by mixed processing of software and hardware. It is to be understood that, when display processing to be executed by the display apparatus in the above-mentioned embodiment is implemented by hardware, the timing for executing each processing procedure is required to be adjusted. In the above-mentioned embodiment, for the sake of convenience of description, details of adjustment of the timings of various kinds of signals accompanying an actual hardware design are omitted.

### Reference Signs List

- 1: tablet supply unit
- 15: cassette controller
- 16: number-of-dispensed-tablets counter
- 2: dispensing unit (manually-dispensed medication processor)
- 23: cell opening/closing controller
- 24: drawer opening/closing detector
- 25: divided tray controller
- 6: prescription control unit
- 61: controller
- 62: storage
- 63: monitor
- 64: input device
- 65: communication IF
- 6A: computer
- 7: barcode reader
- 100: drug dispensing device
- 200: prescription input terminal

## Claims

1. A drug dispensing device (100), which is configured to separately dispense one dose of drugs based on prescription data, the drug dispensing device (100) comprising:
an input device (64) configured to receive input of manufacturing information on a drug to be dispensed given by a worker;
a dispensing unit (2) including a tray (51), which is divided into a plurality of cells, to each of which one dose of the drugs is to be introduced;
a plurality of tablet cassettes (11) each configured to store tablets;
a tablet supply unit (1) configured to automatically dispense the tablets to the tray (51) from each of the plurality of tablet cassettes (11, 12) in accordance with the prescription data;
a monitor (63) configured to display a screen including a manufacturing information input field (904, 905) for supporting work of inputting the manufacturing information on a drug to be dispensed by the worker based on the prescription data and
a controller (61),
**characterized in that**
the controller (61) is configured to:
when a drug other than a tablet to be dispensed from the tablet cassette (11) is to be manually dispensed to the tray (51) of the a dispensing unit (2), display, on the monitor (63), the screen including the manufacturing information input field (904, 905) for supporting, to receive the input of the manufacturing information for each drug to be dispensed,
when one of the plurality of tablet cassettes (11) is to be refilled with tablets, display, on the monitor (63), the screen including the manufacturing information input field (904, 905) for supporting, to receive the input of the manufacturing information, and
store, into a storage (62), the manufacturing information on a drug manually dispensed in work of dispensing a drug to one patient, in association with information for identifying the one patient when the controller (61) determines that the manual-dispensing work is complete for all drugs to be manually dispensed.

2. The drug dispensing device (100) according to claim 1, wherein the tray (51) is configured to receive a tray of a blister pack.

3. The drug dispensing device according to claim 1, further comprising a packaging unit configured to package the drugs introduced to each of the plurality of cells of the tray (51) to an individual drug package.

4. A control program for controlling the drug dispensing device (100) according to claim 1 configured to separately dispense one dose of drugs based on prescription data, the dispensing device (100) comprising: a dispensing unit (2) including a tray (51), which is divided into a plurality of cells, to each of which one dose of the drugs is to be introduced; a plurality of tablet cassettes (11) each configured to store tablets; and a tablet supply unit (1) configured to dispense the tablets to the tray (51) from each of the plurality of tablet cassettes (11, 12) in accordance with the prescription data,
the control program causing a processor of a computer to execute:
input processing of receiving input of manufacturing information on a drug to be dispensed given by a worker;
processing of displaying (S27), on a monitor (63), a screen including a manufacturing information input field (904, 905) , to receive the input of the manufacturing information when a drug other than a tablet to be dispensed from the tablet cassette (11) is to be manually dispensed to the tray (51) of the a dispensing unit (2); and
processing of storing (S30), into a storage (62), the manufacturing information on the drug manually dispensed in work of dispensing a drug to one patient, in association with information for identifying the one patient when the controller (61) determines that the manual-dispensing work is complete for all drugs to be manually dispensed.

5. A computer-readable recording medium having recorded thereon the control program of claim 4.

## Patentansprüche

1. Arzneimittelabgabevorrichtung (100), die dazu aufgebaut ist, eine Dosis von Arzneimitteln auf der Grundlage von Verordnungsdaten separat abzugeben, wobei die Arzneimittelabgabevorrichtung (100) Folgendes umfasst:
eine Eingabevorrichtung (64), die dazu aufgebaut ist, die Eingabe von Herstellerinformationen über ein abzugebendes Arzneimittel zu empfangen, die ein Arbeiter bereitstellt;
eine Abgabeeinheit (2) mit einem Einsatz (51), der in eine Vielzahl von Zellen unterteilt ist, in die jeweils eine Dosis des Medikaments einzubringen ist;
eine Vielzahl von Tablettenkassetten (11), die jeweils zur Aufnahme von Tabletten aufgebaut sind;
eine Tablettenzufuhreinheit (1), die dazu aufgebaut ist, die Tabletten aus jeder der Vielzahl von Tablettenkassetten (11, 12) gemäß den Verordnungsdaten automatisch in den Einsatz (51) abzugeben;
einen Monitor (63), der dazu aufgebaut ist, einen Bildschirm anzuzeigen, der ein Eingabefeld (904, 905) für Herstellerinformationen enthält, um die Arbeit zur Bereitstellung der Eingabe der Herstellerinformationen über ein abzugebendes Medikament durch die Person basierend auf den Verordnungsdaten zu unterstützen, und
eine Steuerung (61),
**dadurch gekennzeichnet, dass**
die Steuerung (61) dazu aufgebaut ist, Folgendes zu tun:
wenn ein anderes Arzneimittel als eine aus der Tablettenkassette (11) abzugebende Tablette manuell in den Einsatz (51) der Abgabeeinheit (2) abzugeben ist, auf dem Monitor (63) den Bildschirm mit dem Eingabefeld (904, 905) für die Herstellerinformationen anzuzeigen, um die Eingabe der Herstellerinformationen für jedes abzugebende Arzneimittel zu unterstützen, und
wenn eine der Vielzahl von Tablettenkassetten (11) mit Tabletten wiederzubefüllen ist, auf dem Monitor (63) den Bildschirm mit dem Eingabefeld (904, 905) für Herstellerinformationen zur Unterstützung anzuzeigen, um die Eingabe der Herstellerinformationen zu empfangen, und
die Herstellerinformationen über ein Arzneimittel, das bei der manuellen Abgabe eines Arzneimittels an einen Patienten abgegeben wird, in einen Speicher (62) in Verbindung mit Informationen zur Identifizierung des einen Patienten zu speichern, wenn die Steuereinheit (61) feststellt, dass die manuelle Abgabe aller manuell abzugebenden Arzneimittel abgeschlossen ist.

2. Arzneimittelabgabevorrichtung (100) nach Anspruch 1, wobei der Einsatz (51) zur Aufnahme eines Einsatzes einer Blisterpackung aufgebaut ist.

3. Arzneimittelabgabevorrichtung nach Anspruch 1, weiter mit einer Verpackungseinheit, die dazu aufgebaut ist, die in jede der Vielzahl von Zellen des Einsatzes (51) eingeführten Arzneimittel als eine individuelle Arzneimittelpackung zu verpacken.

4. Steuerprogramm zum Steuern der Arzneimittelabgabevorrichtung (100) nach Anspruch 1, das dazu aufgebaut ist, eine Dosis von Arzneimitteln basierend auf Verordnungsdaten separat abzugeben, wobei die Abgabevorrichtung (100) Folgendes umfasst: eine Abgabeeinheit (2) mit einem Einsatz (51), die in eine Vielzahl von Zellen unterteilt ist, in die jeweils eine Dosis der Arzneimittel einzubringen ist; eine Vielzahl von Tablettenkassetten (11), die jeweils zum Speichern von Tabletten aufgebaut sind; und eine Tablettenzufuhreinheit (1), die dazu aufgebaut ist, die Tabletten aus jeder aus der Vielzahl von Tablettenkassetten (11, 12) passend zu den Verordnungsdaten an den Einsatz (51) auszugeben,
wobei das Steuerprogramm einen Prozessor eines Computers dazu veranlasst, Folgendes auszuführen:
eine Eingabeverarbeitung des Empfangens von Herstellerinformationen über ein abzugebendes Medikament, die von einer Person eingegeben werden;
eine Verarbeitung (S27) zum Anzeigen eines Bilds mit einem Eingabefeld (904, 905) für Herstellerinformationen auf einem Monitor (63), um die Eingabe der Herstellerinformationen zu empfangen, wenn ein anderes Arzneimittel als eine aus der Tablettenkassette (11) auszugebende Tablette manuell in den Einsatz (51) der Abgabeeinheit (2) auszugeben ist; und
eine Verarbeitung des Speicherns (S30) der Herstellerinformationen über das Arzneimittel, das bei der Abgabe eines Arzneimittels an einen Patienten manuell abzugeben ist, zusammen mit Informationen zum Identifizieren des einen Patienten in einem Speicher (62), wenn die Steuereinheit (61) feststellt, dass die Arbeit der manuellen Abgabe für alle manuell abzugebenden Arzneimittel abgeschlossen ist.

5. Computerlesbares Aufzeichnungsmedium, auf dem das Steuerprogramm nach Anspruch 4 aufgezeichnet ist.

## Revendications

1. Dispositif de distribution de médicaments (100), qui est configuré pour distribuer séparément une dose de médicaments en se basant sur des données de prescription, le dispositif de distribution de médicaments (100) comprenant :
un dispositif d'entrée (64) configuré pour recevoir une entrée d'informations de fabrication sur un médicament à distribuer donnée par un travailleur ;
une unité de distribution (2) comprenant un plateau (51), qui est divisé en une pluralité de cellules, une dose des médicaments étant introduite dans chacune d'entre elles ;
une pluralité de cassettes de comprimés (11) chacune configurée pour stocker des comprimés ;
une unité d'approvisionnement en comprimés (1) configurée pour distribuer automatiquement les comprimés au plateau (51) depuis chacune de la pluralité de cassettes pour comprimés (11, 12) conformément aux données de prescription ;
un moniteur (63) configuré pour afficher un écran incluant un champ d'entrée d'informations de fabrication (904, 905) pour supporter le travail d'entrée des informations de fabrication sur un médicament à distribuer par le travailleur sur la base des données de prescription et
un dispositif de commande (61),
**caractérisé en ce que**
le dispositif de commande (61) est configuré pour :
lorsqu'un médicament autre qu'un comprimé à distribuer depuis la cassette pour comprimés (11) doit être manuellement distribué au plateau (51) d'une unité de distribution (2), afficher, sur le moniteur (63), l'écran incluant le champ d'entrée d'informations de fabrication (904, 905) pour le support, pour recevoir l'entrée des informations de fabrication pour chaque médicament à distribuer,
lorsque l'une de la pluralité de cassettes pour comprimés (11) doit être remplie de comprimés, afficher, sur le moniteur (63), l'écran incluant le champ d'entrée d'informations de fabrication (904, 905) pour le support, pour recevoir l'entrée d'informations de fabrication, et
stocker, dans une mémoire (62), les informations de fabrication sur un médicament manuellement distribué dans un travail de distribution d'un médicament à un patient, en association avec les informations d'identification du patient lorsque le dispositif de commande (61) détermine que le travail de distribution manuelle est terminé pour tous les médicaments à distribuer manuellement.

2. Dispositif de distribution de médicaments (100) selon la revendication 1, dans lequel le plateau (51) est configuré pour recevoir un plateau d'une plaquette.

3. Dispositif de distribution de médicaments selon la revendication 1, comprenant en outre une unité d'emballage configurée pour emballer les médicaments introduits dans chacune de la pluralité de cellules du plateau (51) dans un emballage de médicament individuel.

4. Programme de commande pour commander le dispositif de distribution de médicaments (100) selon la revendication 1 configuré pour séparément distribuer une dose de médicaments en se basant sur des données de prescription, le dispositif de distribution (100) comprenant : une unité de distribution (2) incluant un plateau (51), qui est divisé en une pluralité de cellules, une dose des médicaments étant introduite dans chacune d'entre elles ; une pluralité de cassettes pour comprimés (11) chacune configurée pour stocker des comprimés ; et une unité d'approvisionnement en comprimés (1) configurée pour distribuer les comprimés au plateau (51) depuis chacune de la pluralité de cassettes pour comprimés (11, 12) conformément aux données de prescription,
le programme de commande amenant un processeur d'un ordinateur à exécuter :
un traitement d'entrée de réception d'entrée d'informations de fabrication sur un médicament à distribuer donnée par un travailleur ;
un traitement d'affichage (S27), sur un moniteur (63), d'un écran incluant un champ d'entrée d'informations de fabrication (904, 905), pour recevoir l'entrée des informations de fabrication lorsqu'un médicament autre qu'un comprimé à distribuer depuis la cassette de comprimés (11) doit être distribué manuellement au plateau (51) de l'unité de distribution (2) ; et
un traitement de stockage (S30), dans une mémoire stockage (62), des informations de fabrication sur le médicament distribué manuellement durant le travail de distribution d'un médicament à un patient, en association avec des informations d'identification du patient lorsque le dispositif de commande (61) détermine que le travail de distribution manuelle est terminé pour tous les médicaments à distribuer manuellement.

5. Support d'enregistrement lisible par ordinateur sur lequel est enregistré le programme de commande selon la revendication 4.
